# EUROPEAN PATENT APPLICATION

(11) **EP 3 388 059 A1**
(43) Date of publication of application: **17.10.2018**
(21) Application number: 16872977.0
(22) Date of filing: 06.12.2016
(51) Int. Cl.: A61K 31/164, A61K 9/10, A61K 9/14, A61K 31/7028, A61K 47/36, A61K 47/38, A61P 37/02

(54) **NANOPARTICLE-WATER DISPERSION LIQUID OF GLYCOSPHINGOLIPID**

(30) Priority: 07.12.2015 JP 2015238832
(71) Applicant: Regimmune Corporation, Tokyo, 1030001 (JP)
(72) Inventor: AKIMOTO, Hidetoshi, Tokyo 103-0001 (JP); ISHII, Yasuyuki, Tokyo 103-0001 (JP)
(74) Representative: Müller-Boré & Partner Patentanwälte PartG mbB
(86) International application number: PCT/JP2016/086233
(87) International publication number: WO 2017/099079

(57) **Abstract**

The purpose of the present invention is to provide a nanoparticle dispersion liquid of an α-galactosylceramide-related compound that can be administered in the form of a stable dispersion liquid using water as a solvent. According to the present invention, it is possible to obtain a nanoparticle dispersion liquid, which is not in the form of a liposome dispersion liquid obtained by using a phospholipid or the like, and also to control the size of nanoparticles.

## Description

### TECHNICAL FIELD

The present invention relates to a nanoparticle dispersion solution of a glycosphingolipid.

### BACKGROUND ART

It is known that various kinds of glycosphingolipids exist in a living body. Glycosphingolipids in a living body generally have various sugars β-bonded to ceramides, and exist in the cell membranes of various organs although the abundance of glycosphingolipids varies depending on an organ.

On the other hand, it has been recently reported that a glycosphingolipid with a sugar α-bonded to a ceramide has a strong immunostimulatory action and antitumor activity. α-Galactosylceramide typified by an agelasphin is a glycolipid isolated from an extract of Agelas mauritianus being a kind of sponge, and is known to strongly activate NKT cells (Non-Patent Document 1).

α-Galactosylceramide is taken in antigen presenting cells (APC) typified by dendritic cells (DC) etc., and is then presented on cell membranes by a CD1d protein similar to major histocompatibility complex (MHC) class I molecule. NKT cells are activated by recognizing the thus-presented complex of a CD1d protein and α-galactosylceramide (α-GalCer) using TCR, and various immune reactions are initiated.

α-Galactosylceramide is a glycosphingolipid in which galactose is α-bonded to a ceramide formed with a sphingosine base acylated by a long chain fatty acid. Various related compounds as described below have been heretofore synthesized, and a correlation between the structure of such a compound and activity has been examined. 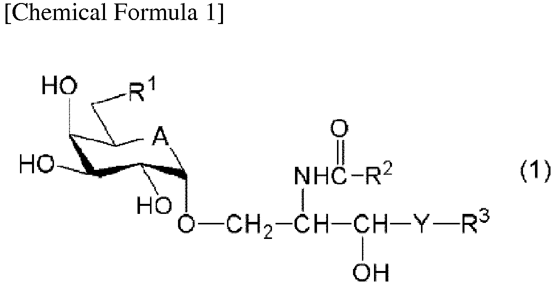 In the formula, R¹ represents a hydrogen atom, a hydroxyl group, an alkyl group having 1 to 7 carbon atoms, an alkoxy group having 1 to 6 carbon atoms or a halogen atom, R² and R³ each independently represent a substituted or unsubstituted hydrocarbon group having 10 to 28 carbon atoms, Y represents -CH2-, -CH(OH)- or -CH=CH-, and A represents O or C.

In recent years, attention has been paid to the functions of such NKT cells, and therapeutic agents containing α-GalCer as an active ingredient have been proposed and developed. However, NKT cells activated by administration of α-GalCer have an action of enhancing IFN-γ production by IFN-γ and NKT cells being cytokines which are useful for cancer treatment, and induce immunostimulatory activity, and the NKT cells produce IL-12 being a cytokine produced by dendritic cells, and simultaneously produce IL-4 being a cytokine that induces an immunosuppressive action, and IL-10 being a cytokine that induces an immunomodulatory action. As a result, there is the problem that the effect of immunostimulatory activity is suppressed, and thus it is difficult to obtain a sufficient effect for cancer treatment.

A glycosphingolipids is almost insoluble in water, and is dissolved using a DMSO solution or a surfactant, or used as a liposome (Patent Document 2) with a phospholipid or the like. However, the state in which a glycosphingolipid exists in the DMSO solution after administration is unknown, and the dosage of the DMSO or surfactant is limited because of its toxicity. In addition, the liposome has a problem in its stability.

### PRIOR ART DOCUMENTS

### PATENT DOCUMENT

Patent Document 1: International Publication No. WO 2005/120574

### NON-PATENT DOCUMENT

Non-Patent Document 1: Science 1997, 278, 1626-1629

### SUMMARY OF THE INVENTION

### PROBLEMS TO BE SOLVED BY THE INVENTION

The present invention has been made in view of the above-described situations, and an object of the present invention for solving the above-described problems is to provide a nanoparticle dispersion liquid of an α-galactosylceramide-related compound that can be administered as a stable dispersion liquid with water as a solvent.

### MEANS FOR SOLVING THE PROBLEMS

The present inventors have extensively conducted studies for solving the above-described problems, and resultantly found that a nanoparticle dispersion liquid can be obtained without forming a dispersion liquid as a liposome using a phospholipid or the like, and the size of nanoparticles can be controlled.

Specifically, the present invention includes:
(1) nanoparticles of a glycosphingolipid;
(2) a nanoparticle-aqueous dispersion liquid of a glycosphingolipid;
(3) the aqueous dispersion liquid according to (2), wherein the aqueous dispersion liquid is a hydrophobic colloidal-aqueous dispersion liquid;
(4) the aqueous dispersion liquid according to (3), wherein the aqueous dispersion liquid is a protective colloidal-aqueous dispersion liquid;
(5) the aqueous dispersion liquid according to any one of (2) to (4), wherein the glycosphingolipid is at least one selected from the group consisting of a compound represented by the formula (1) and a salt thereof: 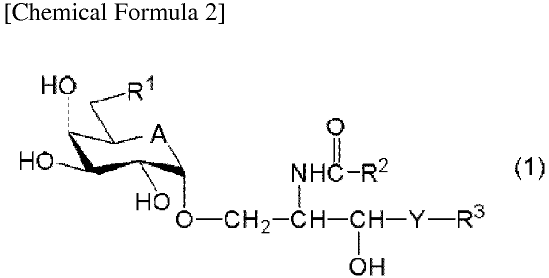 In the formula, R¹ represents a hydrogen atom, a hydroxyl group, an alkyl group having 1 to 7 carbon atoms, an alkoxy group having 1 to 6 carbon atoms or a halogen atom, R² and R³ each independently represent a substituted or unsubstituted hydrocarbon group having 10 to 28 carbon atoms, Y represents -CH2-, -CH(OH)- or -CH=CH-, and A represents O or C.
(6) the aqueous dispersion liquid according to (5), wherein R¹ is a hydroxyl group, an alkyl group having 1 to 4 carbon atoms, or an alkoxy group having 1 to 4 carbon atoms, R² is a substituted or unsubstituted hydrocarbon group having 24 to 28 carbon atoms, and R³ is a substituted or unsubstituted hydrocarbon group having 11 to 15 carbon atoms;
(7) the aqueous dispersion liquid according to (6), wherein R² is an unsubstituted hydrocarbon group having 24 to 28 carbon atoms, R³ is an unsubstituted hydrocarbon group having 11 to 15 carbon atoms, and Y is -CH(OH)-;
(8) the aqueous dispersion liquid according to any one of (2) to (7), wherein the glycosphingolipid is at least one selected from the group consisting of KRN7000, RCAI-56 and RCAI-61;
(9) the aqueous dispersion liquid according to (4), wherein the protective colloid includes a hydrophilic colloid of a polysaccharide and a hydrophobic colloid of a glycosphingolipid;
(10) the aqueous dispersion liquid according to (9), wherein the polysaccharide is at least one selected from inulin, pullulan and hydroxypropyl methylcellulose;
(11) a pharmaceutical including the aqueous dispersion liquid according to any one of (2) to (10) as an active ingredient;
(12) a method for producing a nanoparticle-aqueous dispersion liquid of a glycosphingolipid, the method including dissolving a glycosphingolipid in an organic solvent which can be freely mixed with water, and mixing the solution with water to produce a nanoparticle-aqueous dispersion liquid;
(13) the method for producing a nanoparticle-aqueous dispersion liquid of a glycosphingolipid according to (12), wherein the organic solvent is one selected from ethanol, methanol, acetone, DMSO, n-propanol, isopropanol, tert-butyl alcohol and dimethylformamide, or a mixture of two or more of the organic solvents; and
(14) the method for producing a nanoparticle-aqueous dispersion liquid of a glycosphingolipid according to (12) or (13), wherein the organic solvent and water are used at 50°C or higher.

### ADVANTAGES OF THE INVENTION

There are no commercially available products of glycosphingolipids because it is difficult to control immunostimulation and immunosuppression. A glycosphingolipid aqueous dispersion liquid of the present invention is taken in antigen presenting cells such as dendritic cells, and presented to NKT cells. Control can be performed to take the glycosphingolipid aqueous dispersion liquid in a certain kind of antigen presenting cells by appropriately selecting an administration route and a state (size or dispersion form) of a glycosphingolipid, and therefore immunostimulation and immunosuppression can be controlled in a variety of ways. Thus, the glycosphingolipid aqueous dispersion liquid can be used for treatment of many immune-related diseases.

### BRIEF DESCRIPTION OF THE DRAWINGS

Fig. 1 is a diagram showing the results of administering a glycosphingolipid hydrophobic colloidal solution (iGal) with inulin, α-GalCer (KRN7000) or an α-GalCer liposome (RGI-2001) into the tail vein of a mouse, and measuring the concentration of IL-2 in serum in Example 5.
Fig. 2 is a diagram showing the results of administering iGal, KRN7000 or RGI-2001 into the tail vein of a mouse, and measuring the concentration of IL-4 in serum in Example 5.
Fig. 3 is a diagram showing the results of administering iGal, KRN7000 or RGI-2001 into the tail vein of a mouse, and measuring the concentration of IL-10 in serum in Example 5.
Fig. 4 is a diagram showing the results of administering iGal, KRN7000 or RGI-2001 into the tail vein of a mouse, and measuring the concentration of IFN-γ in serum in Example 5.
Fig. 5 is a diagram showing the results of administering iGal, KRN7000 or RGI-2001 into the tail vein of a mouse, and measuring the concentration of TNF-α in serum in Example 5.

### EMBODIMENTS OF THE INVENTION

Hereinafter, the present invention will be described in detail with preferred embodiments thereof.

A glycosphingolipid for use in the present invention is a derivative or analogue of α-galactosylceramide (KRN7000: (2S, 3S, 4R)-1-O-(α-D-galactosyl)-N-hexacosanoyl-2-amino-1,3,4-octadecanetriol: a compound represented by the following structural formula).

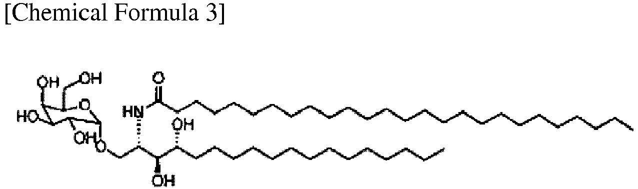

The glycosphingolipid for use in the present invention is preferably a compound represented by the following formula (1), or a salt thereof. 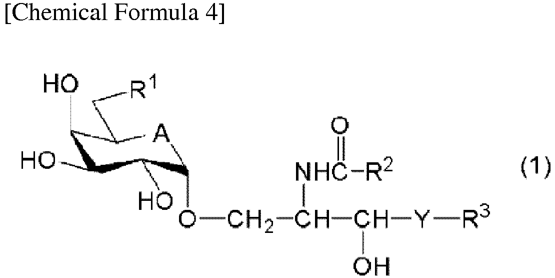 In the formula (1), R¹ represents a hydrogen atom, a hydroxyl group, an alkyl group having 1 to 7 carbon atoms, an alkoxy group having 1 to 6 carbon atoms or a halogen atom, R² and R³ each independently represent a substituted or unsubstituted hydrocarbon group having 10 to 28 carbon atoms (the hydrocarbon group is linear or branched, preferably linear), Y represents -CH2-, -CH(OH)- or -CH=CH-, and A represents O or C.

The glycosphingolipid for use in the present invention is more preferably the compound in which R¹ is a hydroxyl group, an alkyl group having 1 to 4 carbon atoms, or an alkoxy group having 1 to 4 carbon atoms, R² is a substituted or unsubstituted hydrocarbon group having 24 to 28 carbon atoms (the hydrocarbon group is linear or branched, preferably linear), and R³ is a substituted or unsubstituted hydrocarbon group having 11 to 15 carbon atoms.

The glycosphingolipid for use in the present invention is more preferably the compound in which R² is an unsubstituted hydrocarbon group having 24 to 28 carbon atoms, R³ is an unsubstituted hydrocarbon group having 11 to 15 carbon atoms (the hydrocarbon group is linear or branched, preferably linear), and Y is -CH(OH)-.

The salt of the compound represented by the formula (1) is not particularly limited as long as it is pharmaceutically acceptable.

The glycosphingolipid for use in the present invention is most preferably α-galactosylceramide (KRN7000), RCAI-56 or RCAI-61.

The structural formulae of RCAI-56 and RCAI-61 are as shown below, respectively.

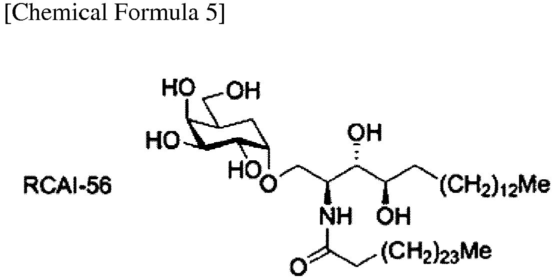

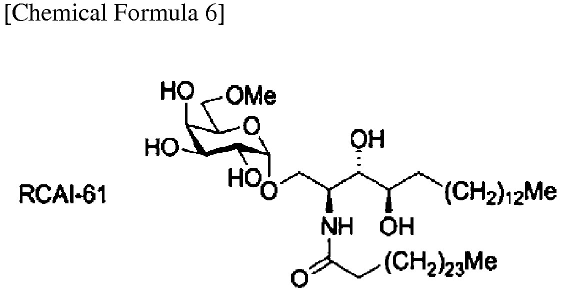

In the present invention, the glycosphingolipids may be used singly, or may be used in combination of two or more thereof.

When a substance that promotes stabilization of glycosphingolipid nanoparticles is used, the ratio between the glycosphingolipid and the stabilization promoting substance is not particularly limited, and for example, the amount of the stabilization promoting substance is 0.5 to 1000 parts by weight, preferably 0.5 to 850 parts by weight, more preferably 0.5 to 500 parts by weight based on 1 part by weight of the sphingolipid.

The glycosphingolipid nanoparticles include a glycosphingolipid singly, or a complex of the glycosphingolipid with a stabilization promoting substance. The size of nanoparticles is not particularly limited as long as it can be a size which allows the nanoparticles to be uniformly dispersed in water, and the size of nanoparticles is preferably 1000 nanometers or less, more preferably 500 nanometers, most preferably 300 nanometers or less. The size of nanoparticles is measured by a dynamic light scattering method.

The substance that promotes stabilization of glycosphingolipid nanoparticles may be any substance as long as it forms a protective colloid with glycosphingolipid nanoparticles, and for example, a polysaccharide is preferable. Inulin, pullulan and hydroxypropyl methylcellulose are especially preferable. These polysaccharides may be used singly, or used in combination of two or more thereof.

The content of the glycosphingolipid in the nanoparticle-aqueous dispersion liquid of a glycosphingolipid is not particularly limited, and may be appropriately set according to a use of the aqueous dispersion liquid, etc., and the content of the glycosphingolipid is, for example, 0.0001 to 5 mg/ml, preferably 0.001 to 5 mg/ml, more preferably 0.01 to 5 mg/ml.

The nanoparticle-aqueous dispersion liquid of a glycosphingolipid can be produced by dissolving a glycosphingolipid in an organic solvent which can be freely mixed with water, and mixing the solution with water.

Examples of the organic solvent which can be freely mixed with water include ethanol, methanol, n-propanol, isopropanol, tert-butyl alcohol, acetone, DMSO and dimethylformamide. Ethanol is preferable. These organic solvents may be used singly, or used in combination of two or more thereof.

The concentration of the glycosphingolipid dissolved in the organic solvent is not particularly limited, and is, for example, 0.01 to 15 mg/ml, preferably 0.05 to 15 mg/ml, more preferably 0.25 to 15 mg/ml.

Here, when the water to be mixed is pure water or the like, a hydrophobic colloidal-aqueous dispersion liquid of a glycosphingolipid is formed. When a polysaccharide that forms a hydrophilic colloid in water is contained, the hydrophilic colloid is combined with the hydrophobic colloid of the glycosphingolipid to form a protective colloid, and therefore the glycosphingolipid can stably exist as nanoparticles of α-galactosylceramide-related compound.

When the glycosphingolipid is hardly soluble in an organic solvent that is mixed with water, the glycosphingolipid can be dissolved by heating. When ethanol is used as a solvent, the temperature is preferably 50°C or higher.

The mixing ratio between the organic solvent in which the glycosphingolipid is dissolved and water may be appropriately set with consideration given to the concentration of the glycosphingolipid in the nanoparticle-aqueous dispersion liquid of a glycosphingolipid, the concentration of the glycosphingolipid dissolved in the organic solvent, or the like, and the mixing ratio is, for example, a ratio such that the amount of water is 3 to 500 parts by weight, preferably 3 to 250 parts by weight, more preferably 3 to 100 parts by weight based on 1 part by weight of the organic solvent in which the glycosphingolipid is dissolved.

The size of nanoparticles can be changed by adjusting the organic solvent used for dissolution, the mixing temperature, the concentration of the solution, or the like. In addition, the sizes of the nanoparticles can be equalized by using a filter etc.

The thus-obtained nanoparticle-aqueous dispersion liquid of a glycosphingolipid can be used as an active ingredient of a pharmaceutical. Control can be performed to take the glycosphingolipid aqueous dispersion liquid in a certain kind of antigen presenting cells by appropriately selecting an administration route and a state (size or dispersion form) of a glycosphingolipid, and therefore immunostimulation and immunosuppression can be controlled in a variety of ways. Thus, the glycosphingolipid aqueous dispersion liquid can be used for treatment of many immune-related diseases.

For example, when used in immunosuppression applications, the glycosphingolipid aqueous dispersion liquid induces NKT and IL-10 producing Tr1 cells as regulatory cells, and has an activity of suppressing activation of helper T cells, and an action of suppressing production of an IgE antibody released from B cells, and therefore the pharmaceutical according to the present invention is effective as a prophylactic or therapeutic agent for allergic diseases caused by an IgE antibody. The IgE antibody is associated particularly deeply with allergic diseases, and by suppressing production (secretion) of the IgE antibody, an effect of preventing or treating type I allergic diseases can be obtained. Examples of the allergic disease associated with the IgE antibody include atopic bronchial asthma, atopic dermatitis, and allergic rhinitis such as allergic rhinitis and hay fever. In the present invention, the prevention of an allergic disease means that not only mammals including humans, that do not have an allergic disease, are prevented from having the disease, but also allergic disease patients (mammals including humans) that have no symptom on a temporary basis are prevented from having symptoms.

In addition, since the glycosphingolipid has an action of suppressing activation of T cells, the pharmaceutical according to the present invention is also effective as a prophylactic or therapeutic agent for diseases such as fulminant hepatitis.

In addition, since the glycosphingolipid has an effect of selectively enhancing the immunosuppressive function of NKT cells, pharmaceuticals containing a nanoparticle-aqueous dispersion liquid of a glycosphingolipid as an active ingredient are also effective as pharmaceuticals having an immunosuppressive function. Specifically, pharmaceuticals containing a nanoparticle-aqueous dispersion liquid of a glycosphingolipid as an active ingredient are also effective as pharmaceuticals for autoimmune diseases such as rheumatism, multiple sclerosis, systemic lupus erythematosus and collagen disease, and pharmaceuticals for GVHD such as rejection in transplantation.

When an immunostimulatory action is selectively exhibited, a complex is formed with a CD1d protein of APC, and presented to NKT cells. NKT cells can suppress production of IL-4 while recognizing the complex through TCR, and selectively and massively producing IFN-γ which is a kind of cytokine that activates the effect of immune cells, among immunomodulatory capabilities of NKT cells themselves. In addition, the nanoparticle-aqueous dispersion liquid of a glycosphingolipid induces production of IL-12 which has an action of enhancing production of IFN-γ by NKT cells. Therefore, the compound (1) according to the present invention or a salt thereof is useful as anticancer agents for inhibition of growth of tumors, and immuno stimulants, and for treatment of cell growth disorders and treatment for correction of Th1/Th2 immuno-balance.

Examples of the cancer treatment target include, but are not limited to, tumors of esophagus, stomach, liver, pancreas, breast, colon, kidney, lung (including small cell lung cancers and non-small cell lung cancers), gall bladder, ovary, testis, bladder, neck, thyroid, prostate and skin (including squamous cell cancers); hematopoietic tumors of lymphatic systems (including leukemia, acute lymphocytic leukemia, acute lymphoblastic leukemia, B cell lymphoma, T cell lymphoma, Hodgkin's lymphoma, non-Hodgkin's lymphoma, hairy cell lymphoma and Burkitt's lymphoma); hematopoietic tumors of bone marrow systems (including acute and chronic myeloid leukemia, myelodysplastic syndrome and promyelocytic leukemia); tumors of mesenchymal origins (including fibrosarcoma and rhabdomyosarcoma); tumors of central nervous systems and peripheral nervous systems (including astrocytoma, neuroblastoma, glioma and schwannoma); and other tumors (including melanoma, seminoma, teratocarcinoma, osteosarcoma, xeroderma pigmentosum and keratoacanthoma, thyroid follicular cancers, and Kaposi's sarcoma).

In addition, cell growth disorders conceptually include familial adenomatous polyposis, psoriasis, benign prostatic hyperplasia, neurofibromatosis, vascular smooth cell growth associated with atherosclerosis, pulmonary fibrosis, arthritis, glomerulonephritis, postoperative stenosis and restenosis.

Examples of the target of administration of the nanoparticle-aqueous dispersion liquid of a glycosphingolipid include mammals such as humans.

When the nanoparticle-aqueous dispersion liquid of a glycosphingolipid is administered to a human, the nanoparticle-aqueous dispersion liquid can be administered as such, or mixed with a pharmacologically acceptable carrier or the like, and orally or parenterally administered safely as a pharmaceutical composition of an orally or parenterally administered agent.

The administration amount of the nanoparticle-aqueous dispersion liquid of a glycosphingolipid varies depending on an age, a body weight, a symptom, a dosage form, an administration method, an administration period and the like, but the administration amount in one patient (adult, body weight: 60 kg) is normally 0.1 to 1 mg/kg of body weight, preferably 0.5 to 1 mg/kg of body weight, more preferably 0.8 to 1 mg/kg of body weight per day. This amount of the nanoparticle-aqueous dispersion liquid can be orally or parenterally administered at one time or in several dosages.

### EXAMPLES

Hereinafter, the present invention will be described in detail by way of examples, but the present invention is not limited to these examples.

### Example 1. Preparation of glycosphingolipid protective colloidal solution using inulin

KRN7000 was weighed in a test tube, 99.5% ethanol was added to adjust the concentration to 4 mg/mL, and the mixture was then heated to 76°C to completely dissolve the KRN7000, so that a glycosphingolipid solution was obtained (the same applies for RCAI-56 or RCAI-61). On the other hand, inulin was weighed in a test tube, sterilized distilled water was added to adjust the concentration to 35 mg/mL, and the inulin was then completely dissolved to obtain an inulin solution. 50 µL of the glycosphingolipid solution was transferred to an additionally provided test tube, 950 µL of the inulin solution was added thereto, and quickly mixed by vortex, the mixture was left standing overnight at room temperature, and an equal amount of pure water was then added to reduce the concentration to 1/2. For the obtained dispersion solution, the average particle size and particle distribution were measured by a dynamic light scattering method (Zetasizer Nano ZS, Malvern). The results of the measurement revealed that an aggregated colloid including polydispersed particles and having a large average particle size was formed when a protective colloid was not used, while a protective colloidal solution including monodispersed particles and having a small average particle size was formed when inulin was used (Table 1). The final components of the glycosphingolipid hydrophilic protective colloidal solution obtained by this method, and the concentrations thereof are as follows: 0.1 mg/mL of glycosphingolipid, 17.5 mg/mL of inulin and 2.5% of ethanol.

### Example 2. Preparation of glycosphingolipid protective colloidal solution using pullulan

KRN7000 was weighed in a test tube, 99.5% ethanol was added to adjust the concentration to 4 mg/mL, and the mixture was then heated to 76°C to completely dissolve the KRN7000, so that a glycosphingolipid solution was obtained (the same applies for RCAI-56 or RCAI-61). On the other hand, pullulan was weighed in a test tube, sterilized distilled water was added to adjust the concentration to 10 mg/mL, and the pullulan was then completely dissolved to obtain a pullulan solution. 50 µL of the glycosphingolipid solution was transferred to an additionally provided test tube, 950 µL of the pullulan solution was added thereto, and quickly mixed by vortex, the mixture was left standing overnight at room temperature, and an equal amount of pure water was then added to reduce the concentration to 1/2. For the obtained dispersion solution, the average particle size and particle distribution were measured by a dynamic light scattering method (Zetasizer Nano ZS, Malvern). The results of the measurement revealed that an aggregated colloid including polydispersed particles and having a large average particle size was formed when a protective colloid was not used, while a protective colloidal solution including monodispersed particles and having a small average particle size was formed when pullulan was used (Table 1). The final components of the glycosphingolipid protective colloidal solution obtained by this method, and the concentrations thereof are as follows: 0.1 mg/mL of glycosphingolipid, 5.0 mg/mL of pullulan and 2.5% of ethanol.

### Example 3. Preparation of glycosphingolipid hydrophilic colloidal solution using hydroxypropyl methylcellulose (HPMC)

KRN7000 was weighed in a test tube, 99.5% ethanol was added to adjust the concentration to 4 mg/mL, and the mixture was then heated to 76°C to completely dissolve the KRN7000, so that a glycosphingolipid solution was obtained (the same applies for RCAI-56 or RCAI-61). On the other hand, HPMC was weighed in a test tube, sterilized distilled water was added to adjust the concentration to 1 mg/mL, and the HPMC was then completely dissolved to obtain a HPMC solution. 50 µL of the glycosphingolipid solution was transferred to an additionally provided test tube, 950 µL of the HPMC solution was added thereto, and quickly mixed by vortex, the mixture was left standing overnight at room temperature, and an equal amount of pure water was then added to reduce the concentration to 1/2. For the obtained dispersion solution, the average particle size and particle distribution were measured by a dynamic light scattering method (Zetasizer Nano ZS, Malvern). The results of the measurement revealed that an aggregated colloid including polydispersed particles and having a large average particle size was formed when a protective colloid was not used, while a protective colloidal solution including monodispersed particles and having a small average particle size was formed when HPMC was used (Table 1). The final components of the glycosphingolipid protective colloidal solution obtained by this method, and the concentrations thereof are as follows: 0.1 mg/mL of glycosphingolipid, 0.5 mg/mL of HPMC and 2.5% of ethanol.

**[Table 1]**

| Protective colloid | Glycosphingolipid | Average particle size (diameter, nm) | Particle size distribution |
|---|---|---|---|
| None | KRN7000 | 1536 | Polydispersed |
| | RCAI-56 | 435 | Polydispersed |
| | RCAI-61 | 683 | Polydispersed |
| Inulin | KRN7000 | 156 | Monodispersed |
| | RCAI-56 | 169 | Monodispersed |
| | RCAI-61 | 298 | Monodispersed |
| Pullulan | KRN7000 | 149 | Monodispersed |
| | RCAI-56 | 174 | Monodispersed |
| | RCAI-61 | 151 | Monodispersed |
| HPMC | KRN7000 | 192 | Monodispersed |
| | RCAI-56 | 380 | Monodispersed |
| | RCAI-61 | 191 | Monodispersed |

### Example 4. Examination of organic solvent used for dissolving glycosphingolipid

Preparation of a hydrophobic colloidal solution of a glycosphingolipid using DMSO as an organic solvent was examined. KRN7000 was weighed in a test tube, DMSO was added to adjust the concentration to 2 mg/mL, and the mixture was heated to 85°C to completely dissolve the KRN7000, so that a glycosphingolipid solution was obtained. On the other hand, inulin was weighed in a test tube, sterilized distilled water was added to adjust the concentration to 35 mg/mL, and the inulin was then completely dissolved to obtain an inulin solution. 200 µL of the glycosphingolipid solution was transferred to an additionally provided test tube, 800 µL of the inulin solution was added thereto, and quickly mixed by vortex, and the mixture was left standing overnight at room temperature. For the obtained dispersion solution, the average particle size and particle distribution were measured by a dynamic light scattering method (Zetasizer Nano ZS, Malvern). The results of the measurement revealed that an aggregated colloid including polydispersed particles and having a large average particle size was formed when a protective colloid was not used, while a protective colloidal solution including monodispersed particles and having a small average particle size of 345.5 nm was formed when inulin was used. The final components of the glycosphingolipid protective colloidal solution obtained by this method, and the concentrations thereof are as follows: 0.4 mg/mL of glycosphingolipid, 28 mg/mL of inulin and 20% of DMSO.

### Example 5. Biological activity test of glycosphingolipid protective colloidal solution using inulin

A biological activity test was conducted for the glycosphingolipid protective colloidal solution prepared in Example 1 (using KRN7000 as a sphingolipid) (hereinafter, referred to as iGal). α-GalCer (KRN7000) solution and an α-GalCer (KRN7000) liposome (hereinafter, referred to as RGI-2001) were used as control substances.

For preparation of the α-GalCer (KRN7000) solution, a dimethyl sulfoxide (DMSO) solution having a concentration of 1 mg/mL was first prepared for α-GalCer (KRN7000), and the DMSO solution was then diluted to 5 times with a phosphate buffer (manufactured by Wako Pure Chemical Industries, Ltd.) containing 0.5% Tween 20 (manufactured by Bio-Rad Laboratories, Inc.), so that a stock solution was obtained. The RGI-2001 was prepared by the method in Ishii et al Front Sci (2008). In this test, each sample was diluted with distilled water for injection or a phosphate buffer so that in administration of the sample into the tail vein in an amount of 100 µL per mouse, the administration amount of α-GalCer was 50 µg/kg of body weight.

100 µL of the prepared iGal solution was injected into the trail vein four times in total at intervals of 7 days in one group of three C57BL/6J female mice (7 weeks old) (about 1 µg of α-GalCer was administered per mouse each time). Using α-GalCer(KRN7000) and RGI-2001 as control substances, a solution of α-GalCer (KRN7000) and RGI-2001 was prepared by a similar method in such a manner that the administration amount of α-GalCer was 50 µg/kg of body weight, and 100 µL of the solution was injected into the tail vein. Immediately before administration of iGal and the control substance and after elapse of 2 hours and 24 hours after administration, 80 µL of blood was collected from the suborbital venous plexus to prepare serum, and the concentration of each cytokine in the serum was measured by a cytokine multi-item simultaneous measurement method (Luminex). In addition, a similar test was conducted using a phosphate buffer (vehicle) as a control in place of each sample.

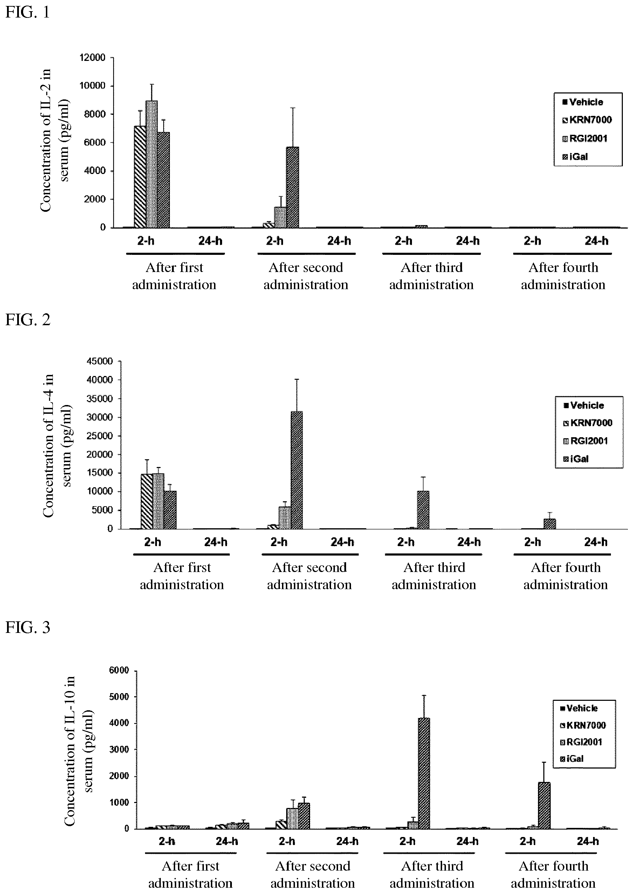

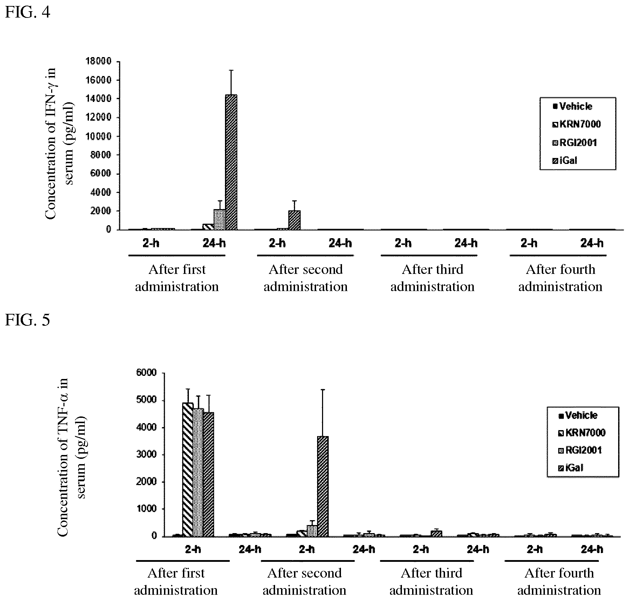

Figs. 1 to 5 show the results of measurement of the concentrations (average values) of IL-2, IL-4, IL-10, IFN-γ and TNF-α in serum immediately before administration, after elapse of 2 hours and 24 hours after first administration, after elapse of 2 hours and 24 hours after second administration, after elapse of 2 hours and 24 hours after third administration and after elapse of 2 hours and 24 hours after fourth administration, and standard deviations (STDEV) thereof. These results showed that iGal more strongly induced production of IFN-γ after single administration than α-GalCer (KRN7000) and RGI-2001. On the other hand, for induction of cytokines IL-2, IL-4, IL-10 and TNF-α after administration performed multiple times, iGal was shown to have a strong and sustainable cytokine induction capability very different from that of α-GalCer (KRN7000) and RGI-2001 because as compared to α-GalCer (KRN7000) and RGI-2001, iGal retained induction activity over multiple times, and remarkably induced production of IL-10 particularly after being administered three times. That is, it has been reveled that the glycosphingolipid protective colloidal solution produced using inulin by forming α-GalCer (KRN7000) into a protective colloid of nanoparticles can more strongly induce production of Th1-type and Th2-type cytokines as compared to α-GalCer (KRN7000) and RGI-2001.

In addition, as a result of a similar test conducted using the glycosphingolipid protective colloidal solution which was produced using a polysaccharide such as pullulan or HPMC by forming α-GalCer (KRN 7000) into a protective colloid of nanoparticles, it has also been reveled that the glycosphingolipid protective colloidal solution can more strongly induce production of Th1-type and Th2-type cytokines as compared to α-GalCer (KRN 7000) and RGI-2001.

## Claims

1. Nanoparticles of a glycosphingolipid.

2. A nanoparticle-aqueous dispersion liquid of a glycosphingolipid.

3. The aqueous dispersion liquid according to claim 2, wherein the aqueous dispersion liquid is a hydrophobic colloidal-aqueous dispersion liquid.

4. The aqueous dispersion liquid according to claim 3, wherein the aqueous dispersion liquid is a protective colloidal-aqueous dispersion liquid.

5. The aqueous dispersion liquid according to any one of claims 2 to 4, wherein the glycosphingolipid is at least one selected from the group consisting of a compound represented by the formula (1) and a salt thereof: 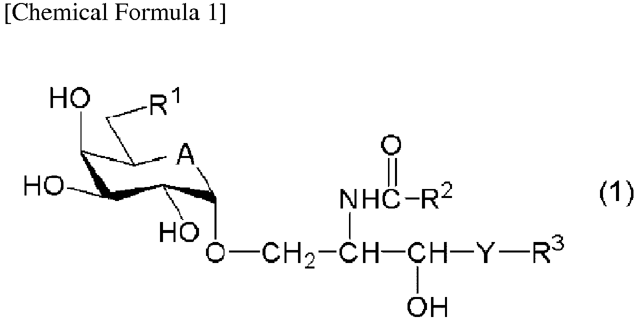 where R¹ represents a hydrogen atom, a hydroxyl group, an alkyl group having 1 to 7 carbon atoms, an alkoxy group having 1 to 6 carbon atoms or a halogen atom, R² and R³ each independently represent a substituted or unsubstituted hydrocarbon group having 10 to 28 carbon atoms, Y represents -CH₂-, -CH(OH)- or -CH=CH-, and A represents O or C.

6. The aqueous dispersion liquid according to claim 5, wherein R¹ is a hydroxyl group, an alkyl group having 1 to 4 carbon atoms, or an alkoxy group having 1 to 4 carbon atoms, R² is a substituted or unsubstituted hydrocarbon group having 24 to 28 carbon atoms, and R³ is a substituted or unsubstituted hydrocarbon group having 11 to 15 carbon atoms.

7. The aqueous dispersion liquid according to claim 6, wherein R² is an unsubstituted hydrocarbon group having 24 to 28 carbon atoms, R³ is an unsubstituted hydrocarbon group having 11 to 15 carbon atoms, and Y is -CH(OH)-.

8. The aqueous dispersion liquid according to any one of claims 2 to 7, wherein the glycosphingolipid is at least one selected from the group consisting of KRN7000, RCAI-56 and RCAI-61.

9. The aqueous dispersion liquid according to claim 4, wherein the protective colloid includes a hydrophilic colloid of a polysaccharide and a hydrophobic colloid of a glycosphingolipid.

10. The aqueous dispersion liquid according to claim 9, wherein the polysaccharide is at least one selected from inulin, pullulan and hydroxypropyl methylcellulose.

11. A pharmaceutical comprising the aqueous dispersion liquid according to any one of claims 2 to 10 as an active ingredient.

12. A method for producing a nanoparticle-aqueous dispersion liquid of a glycosphingolipid, the method comprising dissolving a glycosphingolipid in an organic solvent which can be freely mixed with water, and mixing the solution with water to produce a nanoparticle-aqueous dispersion liquid.

13. The method for producing a nanoparticle-aqueous dispersion liquid of a glycosphingolipid according to claim 12, wherein the organic solvent is one selected from ethanol, methanol, acetone, DMSO, n-propanol, isopropanol, tert-butyl alcohol and dimethylformamide, or a mixture of two or more of the organic solvents.

14. The method for producing a nanoparticle-aqueous dispersion liquid of a glycosphingolipid according to claim 12 or 13, wherein the organic solvent and water are used at 50°C or higher.
